# EUROPEAN PATENT APPLICATION

(11) **EP 4 682 256 A1**
(43) Date of publication of application: **21.01.2026**
(21) Application number: 24770442.2
(22) Date of filing: 21.02.2024
(51) Int. Cl.: C12N 15/113, C12N 5/10, C12N 5/078, C12N 15/12

(54) **COMPOSITION AND USE THEREOF**

(30) Priority: 13.03.2023 JP 2023038193
(71) Applicant: Toagosei Co., Ltd., Minato-ku Tokyo 105-8419 (JP)
(72) Inventor: BAILEYKOBAYASHI, Nahoko, Tokyo 105-8419 (JP); YOSHIDA, Tetsuhiko, Tokyo 105-8419 (JP); BAILEY, Eric Nelson, Tsukuba-shi, Ibaraki 305-0032 (JP)
(74) Representative: Bals & Vogel Patentanwälte PartGmbB
(86) International application number: PCT/JP2024/006140
(87) International publication number: WO 2024/190329

(57) **Abstract**

The composition disclosed herein contains double-stranded RNA that is constituted of a sense strand and an antisense strand. The sense strand includes a main sequence comprising 19 to 23 bases, with the base at the 5'-end being guanine (G) or cytosine (C), and an additional sequence comprising 2 to 4 bases and added to the 3'-end side of the main sequence. The antisense strand includes a sequence complementary to the main sequence, and an additional sequence comprising 2 to 4 bases and added to the 3'-end side of the complementary sequence. The main sequence is constituted of a sequence that is a portion of a base sequence encoding FSTL-1 (follistatin-like 1) and that contains at least a portion of a base sequence encoding a signal peptide region of FSTL-1.

## Description

### Technical Field

The present disclosure relates to a composition and to a method of using this composition. The present disclosure specifically relates to a composition comprising a double-stranded RNA and used for the activation of immune cells. The present application claims priority based on Japanese Patent Application No. 2023-038193 filed on March 13, 2023, and the contents of this application are incorporated in their entirety in the present Description by reference.

### Background Art

Immune checkpoint inhibition therapy is known as an immunotherapeutic method for cancer. This therapeutic method targets so-called "immune checkpoint proteins", for example, PD-1 (Programmed cell death-1), which is known to be a protein involved in a functionality that suppresses an excessive immune response in an organism. In such a therapeutic method, through the use of an inhibitor that inhibits the immune response suppression mechanism, in which an immune checkpoint protein is involved, it is possible to promote attack by the immune system on cancer cells by blocking the "mechanism of avoiding immune system attack (also known as an immune evasion mechanism)" in which the cancer cells are participating.

For example, it has been confirmed that, for some tumors, the immune evasion mechanism can be blocked and the anti-tumor immunoactivity by immune cells can be enhanced by administering to the patient, as an immune checkpoint inhibitor, antibody (anti-PD-1 antibody) relative to PD-1, which is a membrane protein belonging to the immunoglobulin superfamily, or antibody (anti-PD-LA antibody) relative to the ligand of PD-1 known as PD-L1 (Programmed cell death-1 ligand-1; also known as B7-H1), which is expressed in various cells including tumor cells (refer, for example, to WO 2004/004771).

### Citation List

### Patent Literature

Patent Literature 1: WO 2004/004771

### Summary of Invention

### Technical Problem

This all being said, antibody drugs incur high production costs, and it is difficult with antibody drugs to maintain a uniform quality. The present inventors, focusing on nucleic acids, have examined a novel approach to bringing about immune cell activation. Nucleic acid drugs can be mass produced through organic synthesis and facilitate management of a uniform quality.

The main purpose of the present disclosure is to provide a technology that activates immune cells.

### Solution to Problem

The herein disclosed composition is a composition supplied to immune cells in order to activate the immune cells. This composition contains a double-stranded RNA constituted of a sense strand and an antisense strand. The sense strand includes: a main sequence and an additional sequence: the main sequence is composed of 19 to 23 bases, with the base at the 5'-end being guanine (G) or cytosine (C); and the additional sequence is composed of 2 to 4 bases and added to the 3'-end side of the main sequence. The antisense strand includes a sequence complementary to the main sequence and an additional sequence composed of 2 to 4 bases and added to the 3'-end side of this complementary sequence. This main sequence is composed of a sequence that is a portion of a base sequence encoding FSTL-1 (follistatin-like 1) and that contains at least a portion of a base sequence encoding the signal peptide region of FSTL-1.

While the detailed mechanism is unclear, it has been discovered through investigations by the present inventors that a composition with this constitution can activate immune cells.

### Brief Description of Drawings

[Fig. 1]

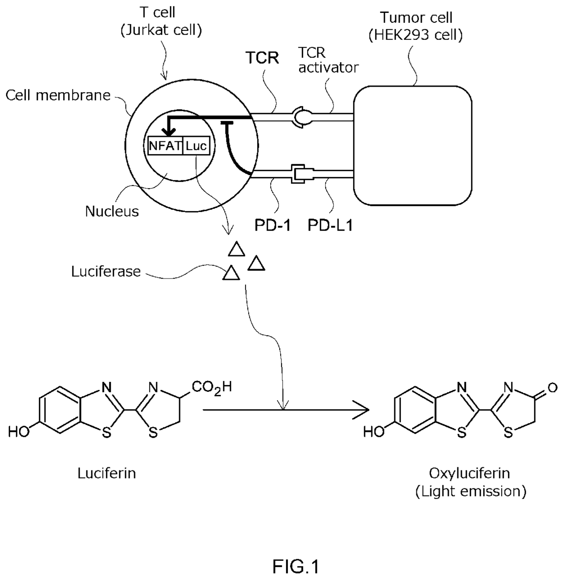

   Fig. 1 is a schematic diagram that shows the outlines of the mechanism of the method for evaluating immune cell activity in the test examples.
[Fig. 2]

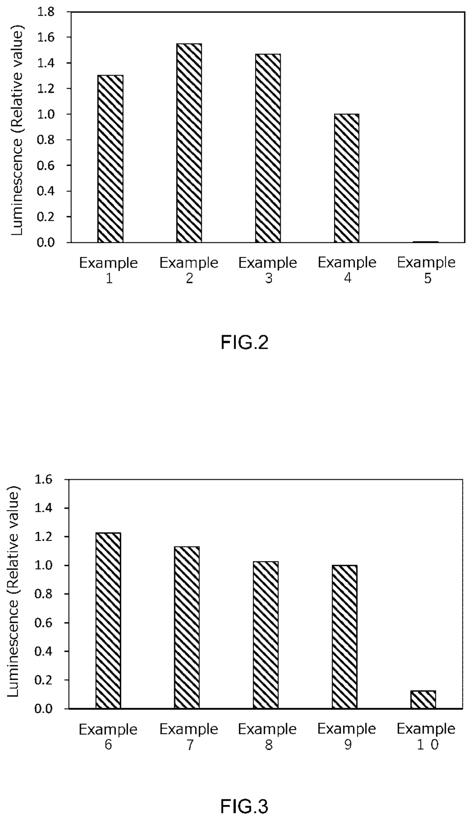

   Fig. 2 is a graph that gives the relative value of the intensity of the luminescence signal measured in Test 1 and Test 2.
[Fig. 3]
   Fig. 3 is a graph that gives the relative value of the intensity of the luminescence signal measured in Examples 6 to 10.

### Description of Embodiments

The herein disclosed technology is described in detail in the following. Matters required for the implementation of the present technology (for example, general matters related to, e.g., methods for the synthesis of polynucleotides), but which are outside those items particularly described in the present Description (for example, the constitution of the double-stranded RNA), can be understood as design matters for the individual skilled in the art based on the conventional art in fields such as cell engineering, physiology, medicine, pharmaceutical science, organic chemistry, biochemistry, genetic engineering, protein engineering, molecular biology, genetics, and so forth. The herein disclosed technology can be implemented based on the contents disclosed in the present Description and the common general technical knowledge in the relevant fields.

In the present Description, "polynucleotide" is a term that denotes a polymer in which a plurality (at least two) of nucleotides are bonded via a phosphodiester bridge, and there is no limitation here with regard to the number of nucleotides. For example, a species containing both a deoxyribonucleotide and a ribonucleotide as nucleotides is also encompassed by "polynucleotide" in the present Description.

In the present Description, "artificially designed polynucleotide" refers to a polynucleotide for which the nucleotide chain (full length) thereof does not exist independently in nature and which is artificially synthesized by chemical synthesis or biosynthesis (i.e., production based on genetic engineering).

In the present Description, "amino acid residue" is a term that, unless specifically indicated otherwise, encompasses the N-terminal amino acid and C-terminal amino acid of the peptide chain.

For the amino acid sequences described in the present Description, the left side is always the N-terminal side and the right side is always the C-terminal side.

In the present Description, "tumor" is a broadly construed term and refers to tumors in general (typically malignant tumors) including carcinomas and sarcomas, or pathologies of the blood or hematopoietic tissues (leukemia, lymphoma, and so forth). In addition, "tumor cells" is synonymous with "cancer cells" and refers to the cells (so-called malignant cells) that form such tumors and that have typically achieved abnormal proliferation that is independent of the surrounding normal tissue. Therefore, unless specifically indicated otherwise, a cell that has been classified as a tumor cell (cancer cell) rather than a normal cell is called a tumor cell regardless of the origin or properties of the cell.

In the present Description, "immune cells" can include immune cells involved in innate immunity and immune cells involved in adaptive immunity. Immune cells involved in innate immunity can be exemplified by neutrophils, macrophages, dendritic cells, natural killer cells (NK cells), mast cells, and so forth. Immune cells involved in adaptive immunity can be exemplified by T cells, B cells, and so forth. The T cells include helper T cells and killer T cells.

In the present Description, insofar as "5‴ and "3‴ designations are not provided, the left side of a base sequence is always the 5'-end side and the right side is always the 3'-end side.

In the present Description, when a numerical range is given by A to B (A and B here are any numerical values), this means, in accordance with the general interpretation, at least A and not more than B (contains the range above A and below B).

The herein disclosed composition contains double-stranded RNA. This double-stranded RNA is constituted of a sense strand and an antisense strand. The sense strand is provided with a main sequence and can also be provided with an additional sequence. The antisense strand contains a sequence complementary to the main sequence of the sense strand. The sense strand and antisense strand hybridize over the main sequence portion to form a duplex. The sense strand and antisense strand can be artificially synthesized polynucleotides.

The main sequence of the sense strand of the herein disclosed double-stranded RNA may be, for example, at least 19 bases and not more than 23 bases, or at least 19 bases and not more than 21 bases, and may be constituted of 19 bases.

The main sequence of the sense strand can be the base sequence of a portion of the base sequence that encodes FSTL-1 and can contain at least a portion of the base sequence that encodes the signal peptide region of FSTL-1 (the base sequence that encodes the signal peptide region of FSTL-1 is also referred to in the present Description as the "FSTL-1-SP base sequence"). As a consequence of this, the main sequence has the same sequence as the sequence of a portion of the mRNA for FSTL-1, and the double-stranded RNA can therefore function as siRNA targeted to FSTL-1. In addition, while the precise mechanism is unclear, the herein disclosed double-stranded RNA, without functioning as siRNA, can function to activate immune cells by contact with the cell membrane surface of immune cells, which is shown in the test examples provided below.

In the present Description, the animal species that is the source of the "FSTL-1" is not particularly limited, but is preferably the same as the animal species of the immune cells to which the herein disclosed composition is to be supplied. For example, when the herein disclosed composition is to be supplied to human-derived immune cells, a base sequence based on the base sequence of human FSTL-1 is favorably used as the main sequence. Human-derived FSTL-1 is described herein as a preferred example, but the present technology may also be applied to FSTL-1 derived from other animal species.

The base sequences of FSTL-1 can be acquired from international databases, for example, from the NCBI (National Center for Biotechnology Information), UniPlot, Ensembl, and so forth databases. Information on the signal peptide region of FSTL-1 can also be acquired from international databases such as these.

Human FSTL-1 is composed of 309 amino acid residues. The signal peptide of human FSTL-1 is an amino acid sequence composed of the 20 amino acid residues shown in SEQ ID NO: 3. This amino acid sequence is the amino acid sequence at positions 1 to 20 of the amino acid sequence that constitutes human FSTL-1. The human FSTL-1-SP base sequence is constituted of the 60 bases given by SEQ ID NO: 4.

The percentage for the FSTL-1-SP base sequence in the main sequence is, using 100% for the whole main sequence, preferably, for example, at least 30%, and may be at least 50%, or at least 75%, or at least 90%, or 100%. Since the FSTL-1-SP base sequence is present on the upstream side of the mRNA, the double-stranded RNA, when functioning as siRNA, can effectively suppress the expression of FSTL-1.

The main sequence typically is constituted of a polynucleotide that is a ribonucleotide polymer. In other words, the main sequence is constituted of RNA. That is, the base sequence of the main sequence is typically indicated using the four characters A (adenine), U (uracil), G (guanine), and C (cytosine), or the four characters a, u, g, and c. However, in the appended sequence listings, uracil may be represented with thymine.

The 5'-end side of the main sequence is preferably guanine or cytosine. The stability of the 5'-end side of the sense strand (i.e., the 3'-end side of the antisense strand) is higher since guanine and cytosine have a stronger binding strength with the complementary strand than do adenine and uracil. In other words, the stability of the 5'-end side of the antisense strand becomes relatively lower. Although the details of the mechanism are not clear, RISC (RNA-induced silencing complex), which is an RNAi-involved protein, tends to preferentially incorporate, between the sense strand and the antisense strand, the strand having the more energetically unstable 5'-end side. Therefore, by having the 5'-end of the main sequence be guanine or cytosine, incorporation of the antisense strand into RISC is facilitated and RNAi can be more favorably induced. As a consequence of this, the double-stranded RNA can favorably function as siRNA.

The 5 bases at the 3'-end side of the main sequence preferably contain at least 60% (i.e., at least 3 bases) adenine and/or uracil and may contain at least 80% (i.e., at least 4 bases) or 100% (i.e., 5 bases). As a consequence, the stability of the 5'-end side of the antisense strand becomes relatively lower than that of the 3'-end side. As a result, the incorporation of the antisense strand into RISC is facilitated and RNAi can be more favorably induced.

The GC content of the whole main sequence (the total percentage of G and C in the whole base sequence that constitutes the main sequence) is not particularly limited, but, for example, may be at least 20% and not more than 60%, is preferably at least 30% and not more than 50%, and may be at least 30% and not more than 45%. The GC content is a parameter related to, inter alia, the binding strength between the antisense strand incorporated into RISC and RNA having the main sequence, the ease of RNA cleavage, and so forth. The RNAi effect can be favorably exerted when the GC content is as indicated in the preceding.

The main sequence, for example, can be either of the following base sequences.
GGGUCCGCGCCGAGGAAGA (SEQ ID NO: 1)
CGCGCCGAGGAAGAGCUAA (SEQ ID NO: 2)
The base sequences shown in SEQ ID NOs: 1 and 2 are both constituted of RNA.

The base sequence given by SEQ ID NO: 1 is the base sequence for positions 50 to 68 of the base sequence that encodes human FSTL-1 (i.e., the sequence from the start codon to the stop codon). Within the base sequence given by SEQ ID NO: 1, the base sequence at positions 1 to 11 is a portion of the FSTL-1-SP base sequence, and the base sequence at positions 12 to 19 is a sequence that is not contained in the FSTL-1-SP base sequence.

The base sequence given by SEQ ID NO: 2 is the base sequence for positions 55 to 73 of the base sequence that encodes human FSTL-1. Within the base sequence given by SEQ ID NO: 2, the base sequence at positions 1 to 6 is a portion of the FSTL-1-SP base sequence, and the base sequence at positions 7 to 19 is a sequence that is not contained in the FSTL-1-SP base sequence.

Double-stranded RNA constituted of the main sequence given by SEQ ID NO: 1 or 2 can activate an immune cell by being supplied to the immune cell (for example, a T cell). For example, NFAT signaling in the immune cell (for example, a T cell) can be enhanced.

The main sequence preferably is selected from within the base sequence that encodes FSTL-1, but, within a range in which the effects of the present technology are accomplished, one base or a plurality of bases (for example, two bases) may be substituted by another base, and/or may be deleted, and/or may be added (inserted).

The sense strand of the herein disclosed double-stranded RNA can be provided with an additional sequence that is added at the 5'-end side or 3'-end side of the main sequence. The additional sequence is preferably added to the 3'-end side of the main sequence. RNAi can be more effectively induced through the addition of the additional sequence.

The additional sequence is constituted of a base sequence that is composed of 2 to 4 bases, and is preferably constituted of a base sequence that is composed of 2 bases. The base sequence constituting the additional sequence is not particularly limited, but favorable examples are TT (thymine · thymine) and UU (uracil · uracil). When the additional sequence is TT, the additional sequence is constituted of DNA.

The additional sequence is constituted of a polynucleotide (dimer, trimer, or tetramer). The polynucleotide constituting the additional sequence may be constructed to contain only ribonucleotide, only deoxynucleotide, or both deoxynucleotide and ribonucleotide. That is, the sense strand and antisense strand may be entirely RNA or may be a chimeric polynucleotide of RNA and DNA. The additional sequence can contain, for example, a modified deoxyribonucleotide, a modified ribonucleotide, or another known nucleotide analog.

The sense strand, for example, is constituted of a base sequence of at least 21 bases and not more than 27 bases and can be constituted of at least 21 bases and not more than 25 bases or at least 21 bases and not more than 23 bases. In a preferred example, the sense strand is constituted of 21 bases composed of 19 bases in the main sequence and 2 bases in the additional sequence. RNAi can be effectively induced by this example.

The antisense strand has a base sequence complementary to the main sequence of the sense strand. As a consequence of this, a double-stranded structure is formed through hybridization of the antisense strand with the sense strand. This complementary base sequence portion is typically composed of a ribonucleotide polymer (RNA).

Like the sense strand, the antisense strand has an additional sequence. The additional sequence is added to the 5'-end side or the 3'-end side of the aforementioned complementary base sequence. In a preferred example, when the additional sequence for the sense strand is added to the 3'-end side of the main sequence, the additional sequence for the antisense strand is then added to the 3'-end side of the aforementioned complementary base sequence.

The constitution of the additional sequence for the antisense strand may be the same as the constitution of the additional sequence for the above-described sense strand. The base sequence of the additional sequence for the antisense strand is typically the same as that for the additional sequence of the hybridizing sense strand, but may be a different base sequence.

The antisense strand, for example, is constituted of a base sequence of at least 21 bases and not more than 27 bases and can be constituted of at least 21 bases and not more than 25 bases or at least 21 bases and not more than 23 bases. In a preferred example, the antisense strand is constituted of a base sequence having the same length as the sense strand and its base sequence, excluding the additional sequence, is constituted of a base sequence complementary to the entire main sequence of the sense strand.

The sense strand and antisense strand constituting the herein disclosed double-stranded RNA can be produced in conformity with general methods of chemical synthesis. For example, synthesis can be carried out using a commercial DNA/RNA automatic synthesizer. In addition, the sense strand and the antisense strand may be synthesized in vitro or in vivo based on genetic engineering techniques. The synthesized sense strand and antisense strand are preferably purified; for example, purification can be carried out by HPLC and so forth.

The herein disclosed double-stranded RNA can be produced, for example, by annealing (hybridizing) the sense strand with the antisense strand. The annealing method may be in accordance with heretofore known methods, and the annealing temperature, cooling rate, and so forth can be adjusted in conformity to the base sequence constituting the main sequence. As an example, annealing can be carried out by mixing equal amounts of the sense strand and the antisense strand in a solvent, heating for 1 minute to 5 minutes at 90°C, and then cooling to 4°C to room temperature. For example, distilled water, pure water, ultrapure water, a buffer (for example, pH 7.4 HEPES-KOH buffer, PBS, and so forth) can be used as this solvent. In order to prevent the admixture of active RNase (RNA-degrading enzyme) in the solvent, solvent is preferably used, for example, that has been subjected to a DEPC treatment, an autoclave treatment, and so forth.

The herein disclosed composition can contain, in addition to the double-stranded RNA that has been described in the preceding, various medicinally (pharmaceutically) acceptable carriers in conformity with the form of use. For example, a carrier that is generally used in drugs as a diluent, excipient, and so forth is preferred for the carrier. Such a carrier may vary as appropriate depending on the use and form of the composition, but typical examples are water, physiological buffer solutions, and various organic solvents. In addition, the carrier, for example, may be an aqueous solution of an alcohol (such as ethanol) at an appropriate concentration, or may be glycerol, or may be a non-drying oil such as olive oil, or may be a liposome. Secondary components that can be incorporated in pharmaceutical compositions can be exemplified by various fillers, extenders, binders, wetting agents, surfactants, dyes, fragrances, transfection reagents and so forth. In addition, a carrier as used in heretofore known drug delivery systems can be incorporated.

The form of the composition is not particularly limited. Typical forms are, for example, liquids, suspensions, emulsions, aerosols, foams, granules, powders, tablets, capsules, and ointments. In addition, for use, for example, for injection, the composition may be freeze-dried or granulated for the purpose of preparing a drug solution by dissolution in, e.g., physiological saline or an appropriate buffer solution (e.g., PBS), immediately before use.

The processes as such of preparing a drug (composition) in various forms using the double-stranded RNA (main component) and various carriers (secondary components) as starting materials may be in accordance with heretofore known methods, and these formulation methods as such do not characterize the present disclosure and detailed explanations are therefore omitted. For example, Comprehensive Medicinal Chemistry, edited by Corwin Hansch, published by Pergamon Press (1990), is an example of a detailed information source regarding formulations.

The herein disclosed composition may contain only one species of the double-stranded RNA constituted of prescribed base sequences, or may contain a plurality of species (at least two species) of double-stranded RNA constituted of base sequences that are different from each other.

The herein disclosed composition can be used, for example, as an immunoadjuvant or immunostimulant to activate immune cells. In addition, because it can promote attack on tumor cells by activating immune cells, for example, it can also be advantageously used as an antitumor agent (anticancer agent).

The present disclosure also provides a method of activating immune cells using the herein disclosed composition. The herein disclosed method contains a preparation step of preparing the herein disclosed composition and a step of supplying the composition to an immune cell.

In the preparation step, for example, the herein disclosed composition may be prepared using a heretofore known method, as described in the preceding.

In the supply step, the herein disclosed composition is supplied to immune cells within an organism (in vivo) or outside an organism (in vitro). The animal species of the immune cells is not particularly limited and can be exemplified by mammals, birds, amphibians, reptiles, fish, and so forth. The animal species of the immune cells is preferably the same as the animal species that is the source of the FSTL-1 that forms the basis of the main sequence of the double-stranded RNA contained in the composition. The type of immune cell is also not particularly limited, but T cells are preferred. While cells other than immune cells may be present at the supply destination of the composition, the composition may be supplied only to immune cells.

The method of administration of the composition may conform to the methods heretofore used to treat animals and is not particularly limited. The composition can be used within an organism (in vivo) using a method and dosage in conformity to its form and purpose. For example, as a liquid agent, it can be administered in a desired amount to the affected area (e.g., malignant tumor tissue, virus-infected tissue, inflammatory tissue, and so forth) of a patient or an individual animal (i.e., an organism) by intravenous, intralymphatic, intramuscular, subcutaneous, intradermal, or intraperitoneal injection. Alternatively, a solid form, e.g., a tablet, or a gel or aqueous jelly, e.g., an ointment, can be administered directly to a prescribed tissue (i.e., for example, an affected area such as a tissue or organ that contains tumor cells, inflammatory cells, and so forth). Alternatively, a solid form such as a tablet can be administered orally. In the case of oral administration, the use of encapsulation or a protective (coating) material to suppress degradation by digestive enzymes in the digestive tract is preferred.

The amount of the composition to be supplied in vivo is not particularly limited, but the amount of the double-stranded RNA per 1 kg of the individual animal can be, for example, at least 0.01 mg, at least 0.05 mg, or at least 0.1 mg. The amount of the double-stranded RNA per 1 kg of the individual animal can be, for example, not more than 10 mg, not more than 5 mg, not more than 2 mg, or not more than 1 mg.

The herein disclosed composition can also be used on eukaryotic cells outside the body (in vitro). Eukaryotic cells in vitro include, for example, various cell masses, tissues, viscera and organs, blood, and lymph fluids that have been extracted from an organism, as well as cell lines and so forth. The double-stranded RNA concentration in vitro is also not particularly limited, but, for example, in the culture solution of the supply target that contains immune cells, the double-stranded RNA concentration can be at least 1 nM, at least 5 nM, or at least 10 nM. The double-stranded RNA concentration in such a culture solution, for example, is advantageously not more than 10 µM and can be not more than 5 µM, not more than 2 µM, not more than 1 µM, or not more than 100 nM.

In the herein disclosed method, the double-stranded RNA may be supplied to the interior of an immune cell by a known transfection method; however, the double-stranded RMA may be supplied to an immune cell without using a known transfection method. In other words, the double-stranded RNA may be supplied so as to come into contact with the immune cell surface (the surface can also include membrane proteins, sugar chains, and so forth that are exposed on the surface). For example, a composition not containing a transfection reagent may be supplied to immune cells and incubation may be carried out without artificially forming pores in the cell membranes of the immune cells. In order to bring the double-stranded RNA into contact with the immune cell surface, the double-stranded RNA may be mixed into the culture solution for the immune cells. The double-stranded RNA preferably comes into direct contact with the immune cell surface without an intervening carrier, etc., rather than coming into indirect contact with the immune cell surface via a carrier such as a transfection reagent. The herein disclosed double-stranded RNA can activate immune cells without being introduced into the cells (i.e., without functioning as siRNA).

Known transfection methods can include, for example, chemical gene transfer methods using cationic molecules (such as commercially available transfection reagents), physical transfer methods such as microinjection and electroporation, and biological gene transfer methods using viruses.

"Immune cell activation" by the herein disclosed composition (or double-stranded RNA) can mean not only direct activation (e.g., enhancement of NFAT signaling) whereby the composition triggers a signal that activates immune cells, but can also include the concept of indirect activation (e.g., suppression of the inhibitory mechanism for NFAT signaling) in which a mechanism that inhibits immune cell-activating signals is suppressed. An example of "immune cell activation" is enhancement of NFAT signaling in T cells.

Specific embodiments of the herein disclosed technology, as has been described in the preceding, are described in the following individual items.

### Item 1:

A composition supplied to an immune cell in order to activate the immune cell, wherein
the composition contains double-stranded RNA that is constituted of a sense strand and an antisense strand,
the sense strand includes:
   a main sequence composed of 19 to 23 bases, with the base at the 5'-end being guanine (G) or cytosine (C); and
   an additional sequence composed of 2 to 4 bases and added to the 3'-end side of the main sequence,
   the antisense strand includes:
      a sequence complementary to the main sequence; and
      an additional sequence composed of 2 to 4 bases and added to the 3'-end side of the complementary sequence, and
      the main sequence is composed of a sequence that is a portion of a base sequence encoding FSTL-1 (follistatin-like 1) and that contains at least a portion of a base sequence encoding a signal peptide region of FSTL-1.

### Item 2:

The composition according to item 1, wherein the main sequence is composed of either of base sequences below:
GGGUCCGCGCCGAGGAAGA (SEQ ID NO: 1);
CGCGCCGAGGAAGAGCUAA (SEQ ID NO: 2).

### Item 3:

The composition according to item 1 or 2, wherein at least 3 bases of the 5 bases on the 3'-end side of the main sequence are adenine (A) and/or uracil (U).

### Item 4:

The composition according to any one of items 1 to 3, wherein the base sequence constituting the additional sequence is thymine · thymine (TT).

### Item 5:

A method of activating an immune cell, wherein the method contains a preparative step of preparing the composition according to any one of items 1 to 4; and a supply step of supplying the composition to the immune cell in vitro.

### Item 6:

The method according to item 5, wherein an animal species of the immune cell is the same as animal species of the FSTL-1.

### Item 7:

The method according to item 5 or 6, wherein the supply step includes bringing the double-stranded RNA into contact with the surface of the immune cell.

Several test examples relating to the herein disclosed technology are described in the following. However, the herein described technology is not limited to or by the description in the test examples.

### <Preparation of double-stranded RNA>

Polynucleotide synthesis was requested from Eurofins Genomics K. K. and polynucleotides having the base sequences shown in SEQ ID NOs: 5 to 8 were obtained. The base sequence of each polynucleotide is shown in Table 1. In each polynucleotide, the "TT" (additional sequence) on the 3'-end side is DNA and the remaining sequence (main sequence) is constituted of RNA. With the obtained polynucleotides, the double-stranded RNAs used in Nos. 1 to 3 shown in Table 1 were respectively prepared by annealing the sense strand with the antisense strand with their complementary sequences. Each of the double-stranded RNAs shown in Nos. 1 to 2 was dissolved in PBS to prepare an RNA solution.

### [Table 1]

**Table 1**

| No. | constitution of double-stranded RNAs | |
|---|---|---|
| 1 | sense strand : | 5' GGGUCCGCGCCGAGGAAGATT 3' (SEQ ID NO: 5) |
| | antisense strand : | 3' TTCCCAGGCGCGGCUCCUUCU 5' (SEQ ID NO: 6) |
| 2 | sense strand : | 5' CGCGCCGAGGAAGAGCUAATT 3' (SEQ ID NO: 7) |
| | antisense strand : | 3' TTGCGCGGCUCCUUCUCGAUU 5' (SEQ ID NO: 8) |

As shown in Table 1, the sense strand of the double-stranded RNA No. 1 is constituted of a main sequence composed of SEQ ID NO: 1 (a sequence including a portion of the base sequence that encodes the signal peptide of FSTL-1) and an additional sequence composed of TT added to the 3'-end side of the main sequence. The sense strand of the double-stranded RNA No. 2 is constituted of a main sequence composed of SEQ ID NO: 2 (a sequence that contains a portion of the base sequence that encodes the signal peptide of FSTL-1) and an additional sequence composed of TT added to the 3'-end side of the main sequence. The antisense strand in each example is constituted of a sequence complementary to the main sequence and an additional sequence composed of TT at the 3'end side of this sequence.

### <Mechanism of the method for evaluating immune cell activity>

Fig. 1 is a schematic diagram that shows an outline of the mechanism underlying the method for evaluating immune cell activity in Test 1 and Test 2 described in the following. In Test 1 and Test 2, Jurkat cells (human T lymphocyte cells) were used and HEK293 cells (human fetal kidney cells) were used as tumor cells. The Jurkat cells (purchased from BPS Bioscience, Inc.) had been constructed to stably express PD-1 and to express luciferase via the NFAT signal pathway. In addition, the Jurkat cells endogenously expressed TCR (T cell receptor) constitutively. The HEK293 cells had been constructed to express TCR activator and PD-L1.

It is generally known that the binding of PD-1 with PD-L1 suppresses the NFAT signal that is induced by the binding of TCR with TCR activator. The NFAT signal is a signal that activates Jurkat cells. That is, Jurkat cells contain a mechanism whereby, when a nearby cell expresses PD-1, the PD-1 binds to the PD-L1, thereby suppressing their own activation.

In Test 1 and Test 2, the Jurkat cells express luciferase via the NFAT signal pathway due to the binding of the TCR expressed by the Jurkat cells to the TCR activator expressed by the HEK293 cells. The expressed luciferase is secreted to the cell exterior (culture medium). The secreted luciferase converts the substrate (luciferin) mixed in the culture medium into oxyluciferin, which is the luminescent substance. The intensity of the luminescence signal in the culture medium is then detected. This can be used as an indicator of Jurkat cell activation. A higher intensity for the luminescence signal indicates a stronger activity.

### [Test 1]

In Test 1, the activity of the Jurkat cells, which are immune cells, was measured when the double-stranded RNA prepared as described above was supplied to the Jurkat cells using a transfection reagent. All of the experiments were performed in triplicate (n = 3, 3 wells for each example). In Test 1, the double-stranded RNA could function as siRNA through the use of the transfection reagent.

### (Example 1)

In Example 1, on the first day, the HEK293 cells were suspended in a culture medium of DMEM (Fujifilm Wako Pure Chemical Industries, Ltd.) + 10% FBS (Hyclone Inc.) + 0.5% penicillin-streptomycin (Invitrogen) in a collagen I-coated 96-well plate, and were seeded at 3.5 × 10⁴ cells/100 µL/well and incubated overnight at 37°C under 5% CO₂.

On the second day, 22 µL of Component A (a reagent containing a vector that causes the overexpression of TCR activator and PD-L1) from the TCR activator/PD-L1 Mammalian Expression kit from BPS Bioscience, Inc. was mixed with 220 µL of Opti-MEM (trademark) to prepare a solution A. In addition, 4.4 µL of Lipofectamine (trademark) 2000 was mixed with 220 µL of Opti-MEM (trademark) to prepare a solution B. Solution A and solution B were then mixed to prepare a solution C, and incubation was carried for 5 minutes at room temperature. The prepared solution C was added to provide 20 µL/well to the wells in which the HEK293 cells were being cultured. This was followed by incubation overnight at 37°C under 5% CO₂.

In addition, the Jurkat cells were suspended in a culture medium (RPMI-1640 (Fujifilm Wako Pure Chemical Industries, Ltd.) + 10% FBS (Hyclone Inc.) + 0.5% penicillin-streptomycin (Invitrogen)), and the Jurkat cells were seeded into 1 well of a 48-well plate for suspended cells to provide 2.8 × 10⁵ cells/400 µL/well. The double-stranded RNA No. 1 of Table 1 was then transfected into the Jurkat cells using Lipofectamine (trademark) RNAiMAX. 4 µM was used for the concentration of the double-stranded RNA in the culture medium when this was done.

On the third day, the prepared Jurkat cells were recovered along with the culture medium to produce a suspension. This suspension was then added to each well of the 96-well plate in which the HEK293 cells had been cultured such that the number of cells of the prepared HEK293 cells and the number of cells of the prepared Jurkat cells were equal (HEK293 cells : Jurkat cells = 1 : 1). This was followed by incubation at 37°C under 5% CO₂ for 16 to 24 hours.

On the fourth day, a luciferase assay was performed using the One-Step Luciferase Assay System, which is a reagent from BPS Bioscience, Inc. for luciferase reporter cell activity measurement. The luminescence signal from luciferase for each well was measured using a microplate luminometer from the Promega Corporation. The measured luminescence signal intensities are reported in Fig. 2. The luminescence signal intensity is the average value of three wells, and a relative value is reported where the luminescence signal intensity value of Example 4, infra, is used as "1". In addition, wells without the introduction of HEK293 cells and Jurkat cells (only culture medium and the luciferase assay reagent) were provided as blank wells. The values reported in Fig. 2 were obtained by subtracting the value of the luminescence signal intensity in the blank from the luminescence signal intensity of the other wells.

### (Example )

Example 2 was performed the same as in Example 1, but changing the double-stranded RNA No. 1 in Example 1 to the double-stranded RNA No. 2 shown in Table 1.

### (Example 3)

An anti-PD-1 antibody (BPS Bioscience, Inc.) was added in place of the double-stranded RNA No. 1 solution. When this was done, the concentration of the anti-PD-1 antibody in the Jurkat cell-containing tubes was brought to 1 µg/mL. Except for this the procedure was the same as in Example 1.

### (Example 4)

The procedure of Example 1 was carried out, except neither the double-stranded RNA nor the anti-PD-1 antibody was added to the Jurkat cell-containing tubes. Thus, Example 4 is an example (Untreatment) in which an additive was not supplied to the Jurkat cells, and is an example that serves as a reference for evaluating Jurkat cell activity. A luminescence signal intensity higher than that of Example 4 indicates that Jurkat cell activation has occurred.

### (Example 5)

The procedure of Example 4 was carried out, but the vector for the TCR activator and PD-L1 was not introduced into the HEK293 cells.

As shown in Fig. 2, the luminescence signal intensity in Examples 1 and 2 was higher than in Example 4, which demonstrates that the Jurkat cells were activated (specifically enhanced NFAT signaling) by transfection of double-stranded RNA No. 1 or No. 2 into the Jurkat cells.

### [Test 2]

In Test 2, the activity of the Jurkat cells, which are immune cells, was measured when the double-stranded RNA prepared as described above was supplied to the Jurkat cells without using a transfection reagent. All of the experiments were performed in triplicate (n = 3, 3 wells for each example).

### (Example 6)

In Example 6, HEK293 cell preparation on the first and second days was performed proceeding as in Example 1 of Test 1. However, seeding of the Jurkat cells on the second day was not carried out. On the third day, the Jurkat cells were suspended in the previously described culture medium used for the Jurkat cells and were dispensed into tubes to give 1.4 × 10⁵ cells/200 µL/tube. A 2 mM RNA solution of double-stranded RNA No. 1 of Table 1 was mixed into the tubes to give a final concentration of 40 µM. The tubes were incubated at 37°C under 5% CO₂ for 1 hour. The Jurkat cell suspension was then added to each well of the 96-well plate in which the HEK293 cells had been cultured such that the number of cells of the prepared HEK293 cells and the number of cells of the prepared Jurkat cells were equal (HEK293 cells : Jurkat cells = 1 : 1). This was followed by incubation at 37°C under 5% CO₂ for 16 to 24 hours. The luminescence signal intensity was subsequently measured on the fourth day proceeding as in Example 1. The measured luminescence signal intensities are reported in Fig. 3. The luminescence signal intensity is the average value of three wells, and a relative value is reported where the luminescence signal intensity value of Example 9, infra, is used as "1". In addition, wells without the introduction of HEK293 cells and Jurkat cells (only culture medium and the luciferase assay reagent) were provided as blank wells. The values reported in Fig. 3 were obtained by subtracting the value of the luminescence signal intensity in the blank from the luminescence signal intensity of the other wells.

### (Example 7)

The same procedure as in Example 7 was followed, with the exception that in Example 7 the double-stranded RNA No. 1 used in Example 6 was changed to the double-stranded RNA No. 2 given in Table 1.

### (Example 8)

The same procedure as in Example 6 was followed, with the exception that an anti-PD-1 antibody (BPS Bioscience, Inc.) was added in place of the double-stranded RNA No. 1 solution with the concentration of the anti-PD-1 antibody in the Jurkat cell-containing tubes being brought to 2 µg/mL.

### (Example 9)

The procedure of Example 6 was carried out, except neither the double-stranded RNA nor the anti-PD-1 antibody was added to the Jurkat cell-containing tubes. Thus, Example 9 is an example (Untreatment) in which an additive was not supplied to the Jurkat cells, and is an example that serves as a reference for evaluating Jurkat cell activity. A luminescence signal intensity higher than that of Example 9 indicates that Jurkat cell activation has occurred.

### (Example 10)

The procedure of Example 9 was carried out, but the vector for the TCR activator and PD-L1 was not introduced into the HEK293 cells.

As shown in Fig. 3, the luminescence signal intensity in Examples 6 and 7 was higher than in Example 9, which demonstrates that the Jurkat cells were activated. Generally, double-stranded RNA cannot penetrate cell membranes due to its negative charge. Due to this, and because a transfection reagent was not used in Examples 6 and 7, it would be difficult to think that the double-stranded RNA No. 1 or No. 2 was taken up into the interior of the Jurkat cells and functioned as siRNA. That is, although the detailed mechanism is unclear, the double-stranded RNA No. 1 or No. 2 is thought to have the function of activating immune cells by contacting the immune cell surface even though it is not taken into the immune cell interior.

Although specific examples of the herein disclosed technology have been described in detail above, these are simply examples and do not limit the claims. The technology described in the claims includes various modifications and alterations of the specific examples provided in the preceding as examples.

## Claims

1. A composition supplied to an immune cell in order to activate the immune cell, wherein
the composition contains double-stranded RNA that is constituted of a sense strand and an antisense strand,
the sense strand comprises:
a main sequence comprising 19 to 23 bases, with the base at the 5'-end being guanine (G) or cytosine (C); and
an additional sequence comprising 2 to 4 bases and added to the 3'-end side of the main sequence,
the antisense strand comprises:
a sequence complementary to the main sequence; and
an additional sequence comprising 2 to 4 bases and added to the 3'-end side of the complementary sequence, and
the main sequence comprises a sequence that is a portion of a base sequence encoding FSTL-1 (follistatin-like 1) and that contains at least a portion of a base sequence encoding a signal peptide region of FSTL-1.

2. The composition according to claim 1, wherein the main sequence comprises either of base sequences below:
GGGUCCGCGCCGAGGAAGA (SEQ ID NO: 1);
CGCGCCGAGGAAGAGCUAA (SEQ ID NO: 2).

3. The composition according to claim 1, wherein at least 3 bases of the 5 bases on the 3'-end side of the main sequence are adenine (A) and/or uracil (U).

4. The composition according to claim 1, wherein the base sequence constituting the additional sequence is thymine · thymine (TT).

5. A method of activating an immune cell, the method comprising:
a preparative step of preparing the composition according to any one of claims 1 to 4; and
a supply step of supplying the composition to the immune cell in vitro.

6. The method according to claim 5, wherein an animal species of the immune cell is the same as an animal species of the FSTL-1.

7. The method according to claim 5, wherein the supply step comprises bringing the double-stranded RNA into contact with the surface of the immune cell.
